# EUROPEAN PATENT APPLICATION

(11) **EP 2 295 105 A1**
(43) Date of publication of application: **16.03.2011**
(21) Application number: 09167334.3
(22) Date of filing: 06.08.2009
(51) Int. Cl.: A61M 16/06

(54) **Respiratory nasal mask with an oxygen port protected by a mobile flap**

(71) Applicant: Air Liquide Medical Systems, 92182 Antony Cedex (FR)
(72) Inventor: Rivetti, Alberto, 25038, ROVATO (BS) (IT); Alberici, Luca, 25128, BRESCIA (IT); Sandoni, Giuseppe, 25124, BRESCIA (IT)
(74) Representative: Pittis, Olivier

(57) **Abstract**

A respiratory mask, in particular a nasal mask, comprising a shell (1) defining an internal chamber (14), a gas inlet orifice (7) in fluid communication with said internal chamber (14) and an oxygen port (8) in fluid communication with said internal chamber (14), **characterized in that** it further comprises a lodging (10) arranged in the shell (1) wall, the oxygen port (8) being arranged in said lodging (10). The mask of the invention can be used in the treatment of respiratory conditions or diseases, such as obstructive sleep apnea or similar.

## Description

The invention concerns a respiratory mask, in particular a nasal mask, with a supplementary gas port, for use in the treatment of respiratory conditions or diseases, such as obstructive sleep apnea.

Nasal masks are commonly used for delivering non-invasive positive pressure ventilation (NPPV) or for nasal continuous positive airway pressure (N-CPAP) therapy of sleep disordered breathing (SDB) conditions, such as obstructive sleep apnea (OSA).

Nasal masks deliver a flow of breathable gas for or to assist patient respiration, especially during the night, i.e., when the patient is sleeping.

Such a mask assembly typically comprises a rigid or semi-rigid hollow shell defining a breathing chamber that receives at least a part of the patient's nose and further comprising a soft face-contacting cushion that comes into contact with the patient face, and a fore-head support and a headgear for correctly positioning, maintaining and/or securing the mask on the head of a patient. An example of a nasal mask of this kind is given by EP-A-462701.

The shell typically receives a gas supply line which delivers a respiratory gas, such as air under pressure, into the breathing chamber of the shell. Further, the soft face-contacting cushion can be made of a soft, resilient elastomeric material that may conform to various facial contours, i.e., the patient face.

The mask assembly is secured to the wearer's head by straps or similar forming a headgear that can be adjusted to pull the mask against the face with sufficient force to achieve a gas tight seal between the mask and the wearer's face as taught by EP-A-462701, EP-A-874667, EP-A-1972357 or WO-A-00/57942.

During the treatment of the patient, it may be necessary, to add additional oxygen to the respiratory flow of gas under pressure, i.e., typically pressurized air, that is delivered to the patient's airways.

Further, it may also be wishable to make some measurements in the mask for the polysomnographic tests.

For doing it, it has been proposed to provide one or several oxygen ports that are used for adding extra oxygen into the mask and/or to plug measurement devices.

The oxygen ports typically comprise a pair of cylindrical connectors molded into the mask frame usually projecting away from the front surface of the frame or located in the lower part of the mask i.e., often close to the bottom of the mask

The oxygen ports typically also include a cap to prevent leakage of air from the mask when the oxygen port in not in use.

However, the existing masks equipped with such ports are not ideal due to the positioning and shape of the ports. Indeed, when the oxygen feeding hoses or the measurement tubes that are connected to them, the resulting arrangement is very cumbersome from the patient.

Further, the position and shape of some ports and closing cap can also lead to dust deposits or similar on or around the port which can lead to hygiene issues.

The problem to be solved is to provide an improved nasal mask architecture allowing extra oxygen to be added to the air flow and measurements to be easily made, solving the problems existing with the masks of the prior art.

The solution of the present invention concerns a respiratory mask comprising a shell defining an internal chamber, a gas inlet orifice in fluid communication with said internal chamber and an oxygen port in fluid communication with said internal chamber, **characterized in that** it further comprises a lodging arranged in the shell wall, the oxygen port being arranged in said lodging.

The mask according to the present invention can further comprise one or more of the following additional features:
- a mobile closing flap is connected to the shell and is able to move between at least a first position wherein said mobile closing flap is, at least partially, covering the lodging, and a second position wherein said mobile closing flap is not covering the lodging.
- the closing flap is mobile in rotation or is pivoting around a connecting element.
- the axis A-A of the oxygen port is parallel to the axis B-B of the gas inlet orifice of the shell.
- the oxygen port comprises a main hollow body with a central hollow passage linking an oxygen inlet situated in the lodging to an oxygen outlet situated in the breathing chamber.
- the lodging is situated in the upper part of the shell between the gas inlet orifice and a front support integral with the shell.
- the shell, the oxygen port and the lodging are molded in one piece.
- the shell comprises an internal chamber with a peripheral border, a cushion being fixed to said peripheral border, said cushion having a central aperture for receiving at least part of the patient's nose, preferably said cushion comprises one or several flexible membranes.
- it is a nasal mask having a shell sized for receiving only at least part of the patient's nose.
- it further comprises a headgear.
- the closing flap is made of a unique piece of plastic, silicone or any other polymeric or elastomeric material.
- the lodging is arranged in the shell wall so as to project toward the internal chamber.

A preferred embodiment of a nasal mask according to the present invention is shown in the enclosed Figures, among which :
- Figure 1 represents a front view of a mask according to the present invention,
- Figures 2 and 3 are enlarged views of the mask of Figure 1,
- Figures 4a to 4c represent the mobile flap of the mask of Figures 1 to 3, and
- Figure 5 shows a lateral view (in cut) of the mask of Figure 1.

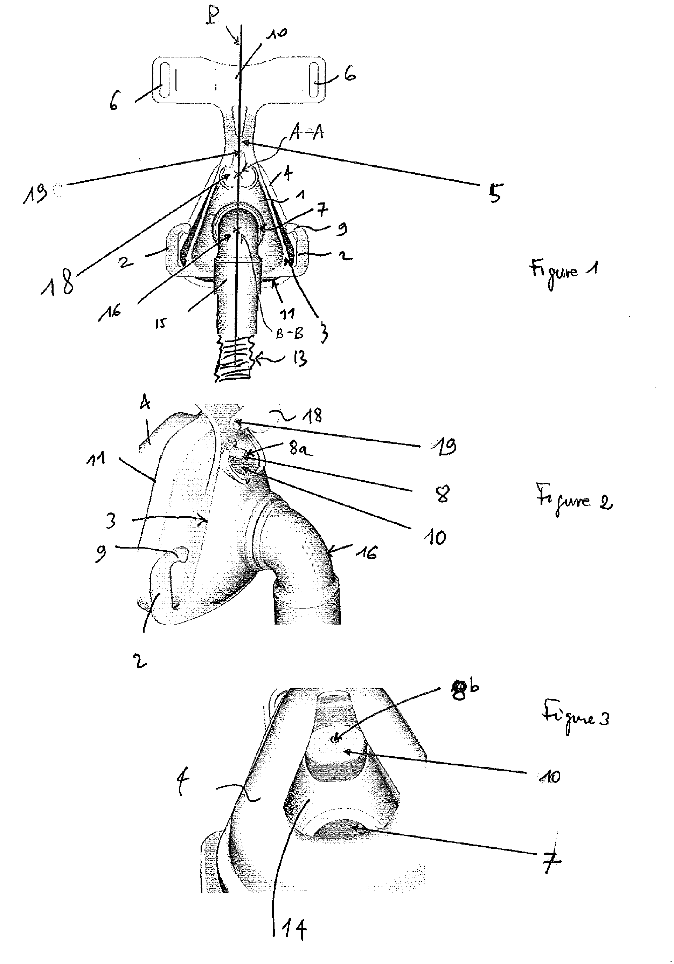

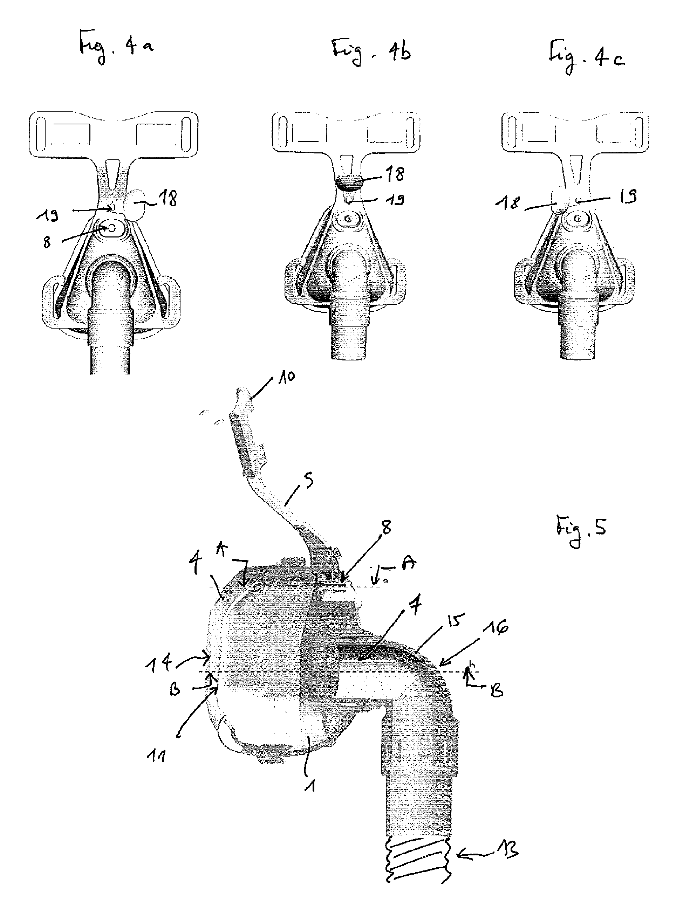

As illustrated in Figures 1 and 5, a respiratory mask according to the present invention comprises a rigid or semi-rigid hollow shell 1 defining an internal breathing chamber 14 (cf.

Fig. 3), wherein respiratory gas, such as air under pressure, is introduced via an inlet port 7 to which is connected a gas feeding line by means of a tubular connector 15.

The gas inlet orifice 7 is arranged at the center of the shell 1 and through its wall thereby allowing air during pressure to be introduced in the breathing chamber.

The shell 1 is preferably made of a polymer material, such as polycarbonate (PC), polypropylene (PP), ABS, nylon or polystyrene (PS), and is configured so as to be able to receive at least a part of the patient's nose. In other words, the patient introduces its nose into the internal volume of the breathing chamber of the shell 1 and breathes the pressurized gas contained therein. The mask further comprises a fore-head support 10 connected to the shell 1 by means of a strip portion 5, and a headgear with straps (not shown) for maintaining the mask in position on the head of the patient. Such a mask structure is well known in the art and disclosed in many documents such as for instance EP-A-1334742, EP-A-462701, EP-A-1985327 or EP-A-956069.

Further, in order to ensure a tight positioning of the nasal mask on the patient's face and to increase the comfort for the patient, the peripheral border or edge 11 of the shell 1 comprises a cushion 4 made of soft, resilient, elastomeric material that comes into contact with the patient face, said cushion 4 having a central aperture 14 for receiving at least part of the patient's nose. More precisely, the border 11 and the cushion 4 have a general triangular or saddle-shape structure so as to match the contours of the nasal region of the patients and hence the cushion 4 comprises, roughly speaking, an upper nasal bridge region, a lower region and cheek regions connecting the nasal bridge and lower regions. In order to improve the seal and tightness, the cushion 4 can comprise one or several membranes, preferably two membranes. Such cushion 4 and shell 1 structures are well-known in the art and taught by many documents, such as EP-A-1334742, EP-A-264772, EP-A-956069, EP-A-874667, US-A-2,931,356 or EP-A-1479406.

The shell 1 is fluidly linked to a gas supply line 13, such as a flexible hose, by means of the tubular hollow connector 15, which delivers a respiratory gas, such as air under pressure, into the breathing chamber of the shell 1. The tubular connector 15 comprises one or several venting outlets, such a several holes 16, for venting to the atmosphere the CO₂-containing gases expired by the patient. The gas supply line 13 is fed with air under pressure by a respiratory device or ventilator (not shown).

Furthermore, several connection means 2, 6 integral with the shell 1 body are provided for fixing the headgear to the shell 1. For instance, as shown in Figures 1 and 5, the frontal support 10 comprises two slots 6 for fixing the straps of the headgear, whereas two headgear-fixing hooks 2 that are arranged on opposite sides of the shell 1, i.e., symmetrically on opposite sides of the shell 1 with respect to the gas inlet 7 and connector 15 thereby allowing the mask to be properly positioned on the patient's nasal region, with a good air tightness and thus obtaining an efficient treatment of sleep disorders, such as OSA or similar. Each headgear-fixing hook 2 is fixed and made integral with the shell 1 body. Preferably, the headgear-fixing hooks 2 are flat parallel portions, but of course, the headgear-fixing hooks 2 can have other forms.

Advantageously, the headgear-fixing hooks 2 and the shell 1 are made of a unique molded structure, preferably a structure made of polymer that is thermo-molded for example. In a preferred embodiment, the shell 1 comprises an external peripheral border 3 arranged around all or only a part of the periphery of shell 1 having a general triangular shape and carrying the headgear-fixing hooks 2, which comprise each a free end 9 comprising a bent portion for retaining the straps of the headgear.

According to the present invention, the shell 1 comprises a lodging 10 in recess arranged in the upper part of the shell 1 body, i.e., into the external wall of the shell 1 between the gas inlet orifice 7 and the string portion 5 linking the front support 10 to the shell 1. The shell 1 has preferably a general triangular tridimensional shape as visible in the Figure 1 for instance.

An oxygen and measurement port 8, i.e. an oxygen connection, is arranged into the lodging 10 as visible on Figure 2 and a closing flap 18 closes the lodging 10, when the oxygen port 8 is not used as shown in Figure 1.

Preferably, the closing flap 18 is connected to the shell 1 by a connecting element 19, such as a screw or similar, but is mobile in rotation or is pivoting around the axis of said connecting element 19 as illustrated in Fig. 4a to 4c, showing the flap 18 in 3 different open positions around the connecting element 19 that constitutes a rotation axis for the flap 8.

In other words, the respiratory nasal mask of the invention comprises an oxygen port 8 situated in the lodging 10 and protected by the closing flap 18.

When the oxygen port 8 is not used, for introducing O₂ in the chamber 14 of the shell 1 or for measurement purposes, the closing flap 18 covers the recess 10 thereby avoiding any dust entrance and deposit. In contrast, when the oxygen port 8 should be used or is used, the closing flap 18 is pivoted/rotated around the axis 19, thereby giving access to the lodging 10 and to the oxygen port 8, on which an oxygen supply line or a measurement device can be plugged in.

The closing flap 18 is preferably made of a unique piece of plastic, silicone or any other polymeric or elastomeric material

As the closing flap 18 remains always connected to the shell 1 thanks to the connecting element 19, for instance an element having the form of a mushroom having its leg integral with the shell 1, said closing flap 18 can never be lost even when the oxygen port is used.

The oxygen port 8 is a classical and standard port comprising a main hollow body with a central passage linking an oxygen inlet 8a situated in the lodging 10 to an oxygen outlet 8b situated in the breathing chamber 14 as shown on Figure 3, for delivering oxygen in said breathing chamber 14. The oxygen port 8 can be also used for making pressure or other measurements into said breathing chamber 14.

Preferably, as illustrated in Figure 5, the axis A-A of the oxygen port 8 is parallel to the axis B-B of the gas inlet orifice 7 of the shell 1. During the night, it happens often that the connector 16 rotates of up to 180° around the axis B-B. As illustrated in Figure 1, the axis A-A of the oxygen port 8 and the axis B-B of the gas inlet orifice 7 are comprised in the plan P of symmetry of the mask 1.

The volume of the lodging 10 is from between 500 mm³ and 2000 mm³. Further, the oxygen port 8 has a length of from about 3 to 15 mm.

The nasal mask of the present invention can be used in a method for treatment of a respiratory disorder or condition, for instance in non-invasive positive pressure ventilation (NPPV) or in a nasal continuous positive airway pressure (N-CPAP) therapy of sleep disordered breathing (SDB) conditions, such as obstructive sleep apnea (OSA).

## Claims

1. Respiratory mask comprising a shell (1) defining an internal chamber (14), a gas inlet orifice (7) in fluid communication with said internal chamber (14) and an oxygen port (8) in fluid communication with said internal chamber (14), **characterized in that** it further comprises a lodging (10) arranged in the shell (1) wall, the oxygen port (8) being arranged in said lodging (10).

2. Mask according to claim 1, **characterized in that** a mobile closing flap (18) is connected to the shell (1) and is able to move between at least:
- a first position wherein said mobile closing flap (18) is, at least partially, covering the lodging (10), and
- a second position wherein said mobile closing flap (18) is not covering the lodging (10).

3. Mask according to any one of the previous claims, **characterized in that** the closing flap (18) is mobile in rotation or is pivoting around a connecting element (19).

4. Mask according to any one of the previous claims, **characterized in that** the axis (A-A) of the oxygen port (8) is parallel to the axis (B-B) of the gas inlet orifice (7) of the shell (1).

5. Mask according to any one of the previous claims, **characterized in that** the oxygen port (8) comprises a main hollow body with a central hollow passage linking an oxygen inlet (8a) situated in the lodging (10) to an oxygen outlet (8b) situated in the breathing chamber (14).

6. Mask according to any one of the previous claims, **characterized in that** the lodging (10) is situated in the upper part of the shell (1) between the gas inlet orifice (7) and a front support (5, 10) integral with the shell (1).

7. Mask according to any one of the previous claims, **characterized in that** the shell (1), the oxygen port (8) and the lodging (10) are molded in one piece.

8. Mask according to any one of the previous claims, **characterized in that** the shell (1) comprises an internal chamber (14) with a peripheral border (11), a cushion (4) being fixed to said peripheral border (11), said cushion (4) having a central aperture for receiving at least part of the patient's nose, preferably said cushion (4) comprises one or several flexible membranes.

9. Mask according to any one of the previous claims, **characterized in that** it is a nasal mask having a shell (1) sized for receiving only at least part of the patient's nose.

10. Mask according to any one of the previous claims, **characterized in that** it further comprises a headgear.

11. Mask according to any one of the previous claims, **characterized in that** the closing flap (18) is made of a unique piece of plastic, silicone or any other polymeric or elastomeric material.

12. Mask according to any one of the previous claims, **characterized in that** the lodging (10) is arranged in the shell (1) wall so as to project toward the internal chamber (14).

13. Mask according to any one of the previous claims, **characterized in that** the axis (A-A) of the oxygen port (8) and the axis (B-B) of the gas inlet orifice (7) are comprised in the plan (P) of symmetry of the mask.
